# FASCICULE DE BREVET EUROPEEN

(11) **EP 1 825 349 B1**
(45) Date de publication et mention de la délivrance du brevet: **14.03.2012**
(21) Numéro de dépôt: 05826012.6
(22) Date de dépôt: 13.12.2005
(51) Int. Cl.: G06F 3/00, A61B 5/117

(54) **PROCEDE DE RECHERCHE D'INFORMATIONS DANS UNE BASE DE DONNEES**
VERFAHREN ZUR DATENSUCHE IN EINER DATENBANK
METHOD FOR DATA SEARCH IN A DATABASE

(30) Priorité: 13.12.2004 FR 0413210
(43) Date de publication de la demande: 29.08.2007
(73) Titulaire: Morpho, 75015 Paris (FR)
(72) Inventeur: WELTI, Paul, F-75015 Paris (FR); FONDEUR, Jean-Christophe, F-92300 Levallois Perret (FR)
(74) Mandataire: Lavialle, Bruno François Stéphane
(86) Numéro de dépôt international: PCT/FR2005/003112
(87) Numéro de publication internationale: WO 2006/064119

(56) Documents cités:
- WO-A-94/08311
- US-A1- 2003 108 241
- US-A1- 2003 165 270
- US-A1- 2004 176 991
- KIAN KANG WU ET AL: "IDENTIFYING FACES USING MULTIPLE RETRIEVALS" IEEE MULTIMEDIA, IEEE COMPUTER SOCIETY, US, vol. 1, no. 2, 21 juin 1994 (1994-06-21), pages 27-38, XP000448660 ISSN: 1070-986X cité dans la demande
- PICARD R W ET AL: "Modeling user subjectivity in image libraries" PROCEEDINGS OF THE INTERNATIONAL CONFERENCE ON IMAGE PROCESSING (ICIP) LAUSANNE, SEPT. 16 - 19, 1996, NEW YORK, IEEE, US, vol. VOL. 2, 16 septembre 1996 (1996-09-16), pages 777-780, XP002351026 ISBN: 0-7803-3259-8 cité dans la demande

## Description

La présente invention concerne un système informatique mettant en oeuvre un procédé de recherche d'informations dans une base de données, et un procédé de recherche dans une base de données.

### ARRIERE PLAN DE L'INVENTION

Après une agression, les enquêteurs tentent généralement d'identifier l'agresseur en soumettant à la victime ou à des témoins des portraits d'individus enregistrés dans une base de données des services de police afin que la victime ou les témoins y reconnaissent l'agresseur. Un autre mode d'identification consiste à reconstituer le visage de l'agresseur par la réalisation d'un portrait robot avec le concours de la victime ou des témoins. Or, il arrive que la victime ou les témoins refoulent, oublient, taisent par peur ou n'aient pas perçu consciemment le visage de l'agresseur, rendant ainsi impossible l'identification de l'agresseur par ces procédés. En outre, la consultation des bases de données de portraits ou la réalisation d'un portrait robot sont des opérations relativement longues et demandant une attention soutenue de la part de la victime généralement bouleversée ou en état de choc, voire blessée. Ces opérations sont donc particulièrement pénibles pour la victime mais également pour les témoins qui peuvent également avoir été émotionnellement touchés.

Le document WO-A-94/08311 décrit ainsi un procédé de recherche dans une base de portraits à des fins policières, dans lequel des portraits sont sélectionnés en fonction d'une caractéristique jugée importante par le témoin.

Il est par ailleurs connu du document US-A-2003/108241 un procédé de consultation d'une base de données préalablement triées en fonction d'un lien entre les données et l'état émotionnel d'un utilisateur habituel, la consultation comprenant, au début d'une consultation de la base de données, l'étape de détecter au moins un paramètre physiologique de l'utilisateur représentatif de l'état émotionnel de celui-ci pour présenter ensuite à celui-ci des données correspondant à cet état émotionnel. La mise en oeuvre de ce procédé suppose d'avoir préalablement affecté aux données un paramètre représentatif de l'état émotionnel en fonction de l'utilisateur, la recherche étant effectuée sur la base de ce paramètre. Ceci est inapplicable pour faire une recherche dans la base de données lorsque l'utilisateur est pour la première fois confronté à cette base de données. Ce procédé est en outre inutilisable si de nombreux utilisateurs sont susceptibles d'utiliser cette base.

Dans une approche similaire, le document US-A-2003/165270 propose de marquer des images présentées à un observateur en fonction de la réaction de l'observateur visionnant ces images.

Il est en outre connu du document "Identifying faces using multiple retrievals" (KIAN KANG WU et al - IEEE Multimedia - IEEE Computer Society - US - Vol 1 No 2 - 21 juin 1994) un procédé de recherche dans une base de données utilisant des paramètres pondérés en fonction de leur importance et du document "Modeling user subjectivity in image libraries" (PICARD - Minutes de la Conférence Internationale de Traitement de l'image ICIP - Lausanne - IEEE - US - vol. 2 - 16 septembre 1996) décrit un procédé d'évaluation de la similarité de deux images.

### OBJET DE L'INVENTION

Il serait donc intéressant de disposer d'un moyen qui permettrait de faciliter et optimiser la recherche d'informations dans une base de données notamment pour accélérer la reconnaissance d'un individu.

### BREVE DESCRIPTION DE L'INVENTION

A cet effet, on prévoit, selon l'invention, un système informatique comportant un serveur qui comprend des moyens de calcul, héberge une base de données et est associé à des moyens de présentation de données de la base de données et à un moyen de mesure d'un paramètre physiologique d'un utilisateur, le système informatique étant agencé pour mettre en oeuvre un procédé de recherche d'informations dans la base de données, le procédé comprenant les étapes de :
- présenter à un observateur au moins une des données et mesurer au moins un paramètre physiologique de l'observateur,
- en présence d'une variation du paramètre physiologique supérieur à un seuil prédéterminé, présenter une donnée ayant une relation de similarité avec la donnée précédemment présentée à l'origine de la variation.

La caractéristique physiologique à laquelle les données sont relatives peut être la morphologie, le visage, la voix... et son observation peut nécessiter la mobilisation d'un ou plusieurs sens de l'observateur. Le paramètre physiologique mesuré peut être le rythme cardiaque, un paramètre lié au flux sanguin comme la saturation du sang en oxygène ou la vasodilatation, le flux salivaire, une réaction musculaire telle qu'une brusque contraction ou un tremblement, une émission de sueur... Ainsi, la variation du paramètre physiologique de l'observateur va constituer une réaction inconsciente ou à tout le moins involontaire de celui-ci à la présentation de la donnée et va permettre d'identifier relativement rapidement les données proches des informations recherchées. Le paramètre physiologique n'est pas un champ de la base de données servant de paramètre de recherche mais un indice d'un impact émotionnel ou d'un intérêt de la donnée pour l'observateur. Il est ensuite recherché une donnée ayant une relation, plus particulièrement une similarité, avec la donnée préalablement présentée (la donnée recherchée a en elle-même une relation avec la donnée initialement présentée, c'est-à-dire que les données ont des contenus présentant une relation : il ne s'agit pas d'une relation entre des paramètres attachés aux données dans la base de données). La prise en considération de l'indication que constitue la variation du paramètre physiologique permet donc d'accélérer la recherche d'informations. Ce procédé permet en outre de limiter le questionnement de l'observateur et ne suscite pas l'impression chez celui-ci de subir un interrogatoire. Ce procédé permet en outre de limiter les explications à dispenser à l'observateur quant au fonctionnement de la présentation.

L'invention a également pour objet un procédé conforme à la revendication 10.

D'autres caractéristiques et avantages de l'invention ressortiront à la lecture de la description qui suit d'un mode de mise en oeuvre particulier non limitatif de l'invention.

### BREVE DESCRIPTION DES DESSINS

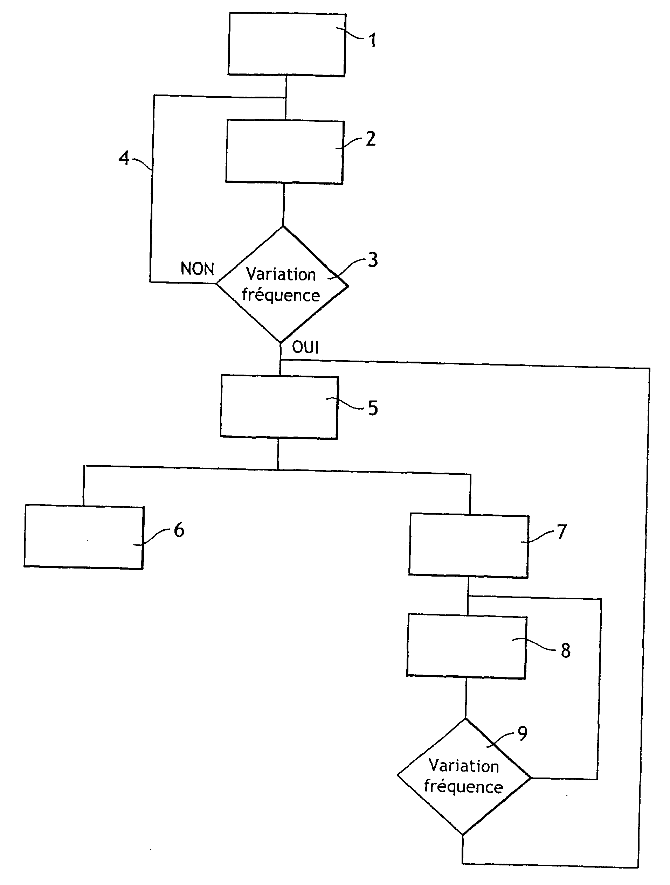

Il sera fait référence à la figure unique annexée, constituée d'un diagramme par blocs illustrant un mode de mise en oeuvre du procédé conforme à l'invention.

### DESCRIPTION DETAILLEE DE L'INVENTION

En référence à la figure, le procédé de l'invention est ici décrit en relation avec la reconnaissance d'un individu par un observateur à partir d'informations ou de données contenues dans une base de données et relatives à une même caractéristique physiologique de différents individus. La caractéristique physiologique est ici le visage et les données sont donc des portraits de ces individus.

La base de données est hébergée sur un serveur informatique comportant des moyens de calcul et associé à un écran de visualisation.

Le procédé de l'invention ici décrit est basé sur la prise en considération d'un paramètre physiologique pour naviguer dans la base de données. Le paramètre physiologique en question est ici la fréquence cardiaque en partant du principe qu'une accélération de la fréquence cardiaque peut résulter d'une augmentation de la tension émotionnelle de l'observateur ou d'un stress de celui-ci provoqué par la vision d'un des portraits. Pour être prise en considération, cette augmentation devra être supérieure à un seuil reflétant une variation de fréquence cardiaque qui ne serait pas liée à une contrainte émotionnelle mais à un effort pour changer de posture par exemple. Le ou les paramètres physiologiques peuvent également être choisis dans la mesure où ceux-ci sont révélateurs d'une émotion, d'une réaction ou d'un comportement particulier lié à l'examen d'une donnée (marquant par exemple l'intérêt).

Le procédé débute par une étape de calibration 1 durant laquelle une mesure de la fréquence cardiaque est réalisée sur l'observateur au repos afin de déterminer un niveau nominal de la fréquence cardiaque compte tenu des circonstances. La mesure est réalisée, de façon connue en soi, par exemple au moyen d'un pulsemètre.

Le procédé se poursuit par une présentation 2 des portraits à l'observateur tandis que la fréquence cardiaque de celui-ci est mesurée et que la direction de son regard est détectée, de façon connue en elle-même, par exemple au moyen d'un oculomètre.

Les portraits sont par exemple affichés sur l'écran de visualisation par groupes.

En l'absence d'une variation de la fréquence cardiaque supérieure au seuil prédéterminé et si l'observateur ne fait pas de remarques particulières, le groupe de portraits suivants est affiché (étape 3).

En présence d'une variation de la fréquence cardiaque supérieure au seuil prédéterminé (étape 4), le portrait regardé au moment de la variation de la fréquence cardiaque est déterminé à partir de la direction du regard de l'observateur (étape 5). En outre, la détection du regard de l'observateur peut permettre d'identifier dans le portrait ainsi déterminé une partie d'intérêt particulier pour l'observateur soit en mesurant et en comparant des durées d'examen des différentes parties de ce portrait soit parce que la vision de cette partie a été immédiatement suivie par la variation de fréquence cardiaque.

Dans le cas ou le défilement des groupes de portraits est automatique, le temps d'affichage de chaque groupe de portraits doit être suffisant pour que la variation de la fréquence cardiaque puisse avoir lieu le cas échéant.

Le procédé se poursuit alors par une étape de notation 6 du portrait en fonction par exemple :
- d'un degré d'attention de l'observateur pour le portrait en question et notamment le nombre de fois où le regard s'est porté sur le portrait, ou la durée d'observation du portrait,
- d'une caractéristique de la variation et notamment l'ampleur de celle-ci, comme la fréquence cardiaque maximale atteinte ou l'accélération du rythme cardiaque.

Il est ensuite procédé à une recherche 7 et à une présentation 8 de portraits ayant une relation avec le portrait à l'origine de la variation de la fréquence cardiaque de l'observateur. Cette relation est une relation de similarité entre les portraits. Cette relation de similarité peut porter de façon prépondérante sur la partie d'intérêt s'il y en a une. Par exemple, si la fréquence cardiaque de l'observateur a augmenté lorsque ce dernier a aperçu une barbe particulière sur le portrait déterminé, les portraits qui seront par la suite présentés porteront une barbe similaire. Il en serait bien entendu de même pour les yeux, la chevelure, le nez, la bouche, une cicatrice... Ceci suppose évidemment que la base de données soit organisée en conséquence. Les portraits à présenter peuvent en outre être déterminés en temps réel ou masqué par analyse en composantes principales pour trouver des points communs entre les portraits à présenter et le portrait à l'origine de la variation de la fréquence cardiaque de l'observateur. La relation de similarité peut prendre en compte les diverses parties d'un visage de façon pondérée pour tenir compte de l'importance relative de ces parties dans la reconnaissance d'un individu par un autre.

En présence d'une nouvelle variation de la fréquence cardiaque (étape 9), les étapes précédentes 5 à 8 sont recommencées.

De nouveaux portraits similaires sont présentés tant que l'observateur n'a pas formellement identifié l'individu recherché. Lorsque tous les portraits similaires de la base de données ont été présentés ou au bout d'un temps déterminé sans qu'un individu n'ait été identifié, une liste de candidats les plus probables est établie en fonction des notes attribuées. Ces candidats peuvent être ceux dont le portrait a déclenché une variation de rythme cardiaque ou des portraits similaires.

En variante, il est possible d'interrompre la présentation lorsqu'un des portraits reçoit une note supérieure à un seuil prédéterminé.

Bien entendu, l'invention n'est pas limitée au mode de réalisation décrit et on peut y apporter des variantes de réalisation sans sortir du cadre de l'invention tel que défini par les revendications.

En particulier, le procédé de recherche n'est pas limité à la recherche d'informations sur des visages mais peut également concerner des silhouettes, des images de lieux ou d'objets. En outre, le procédé de recherche n'est pas limité à la recherche d'informations visuelles mais faisant appel à d'autres sens et notamment à l'ouïe pour des informations auditives.

Il va de soi que d'autres paramètres physiologiques peuvent être utilisés en particulier si une variation de ceux-ci peut être révélatrice d'un stress : un paramètre lié au flux sanguin comme la saturation du sang en oxygène, la vasodilatation ou la pression sanguine ; le flux salivaire ; une réaction musculaire telle qu'une brusque contraction ou un tremblement ; une émission de sueur (réponse électrodermale) ; le temps de réponse à une question suivant la présentation d'une information ; le rythme respiratoire ; la voix (tension ou tremblement, inflexion) ; une piloérection ; le diamètre pupillaire ; l'expression du visage (le mouvement des yeux ou une mimique peuvent modifier l'expression du visage, un réflexe d'occlusion des paupières) ; une posture (un réflexe de protection peut modifier la posture de l'observateur, comme un réflexe stapédien) ; l'activité cérébrale (activation d'une zone particulière du cerveau)... Plusieurs paramètres physiologiques peuvent en outre être simultanément surveillés pour prendre en considération leurs variations.

La détection du regard est facultative notamment lorsque les images sont présentées une par une à l'observateur.

En variante, il est possible de prévoir une étape d'évaluation de la fiabilité de la recherche en comparant selon au moins un critère de ressemblance préétabli les informations ayant une note supérieure à un seuil prédéterminé.

## Revendications

1. Système informatique comportant un serveur qui comprend des moyens de calcul, héberge une base de données et est associé à des moyens de présentation de données de la base de données et à un moyen de mesure d'un paramètre physiologique d'un utilisateur, le système informatique étant agencé pour mettre en oeuvre un procédé de recherche d'informations dans la base de données, **caractérisé en ce que** le procédé comprend les étapes de :
- présenter à un observateur au moins une des données et mesurer au moins un paramètre physiologique de l'observateur,
- en présence d'une variation du paramètre physiologique supérieur à un seuil prédéterminé, présenter une donnée ayant une relation de similarité avec la donnée précédemment présentée à l'origine de la variation.

2. Système selon la revendication 1, **caractérisé en ce qu'**il comprend une étape préalable de calibration dans laquelle une mesure du paramètre physiologique est réalisée pour déterminer un niveau nominal de celui-ci.

3. Système selon la revendication 1, **caractérisé en ce que**, les données étant des images, le procédé comprend l'étape de détecter sur l'image au moins une partie d'intérêt pour l'observateur par un moyen de détection du regard de l'observateur, la relation existant entre l'image précédemment présentée et l'image présentée après la détection de la variation du paramètre physiologique portant sur cette partie d'intérêt.

4. Système selon la revendication 3, **caractérisé en ce que** la partie d'intérêt est détectée en mesurant une durée d'examen de cette partie.

5. Système selon la revendication 3, **caractérisé en ce que** la partie d'intérêt est détectée en identifiant une corrélation entre une vision de cette partie et la
variation du paramètre physiologique.

6. Système selon la revendication 3, **caractérisé en ce que** les images sont présentées par groupe et **en ce que** le procédé comprend l'étape de détecter une direction du regard de l'observateur lors de la présentation des images.

7. Système selon la revendication 1, **caractérisé en ce qu'**il comprend une étape de notation de l'information ayant déclenché la variation du paramètre physiologique au cours de laquelle est attribuée à l'information une note prenant en compte un degré d'attention de l'observateur pour le portrait et/ou une caractéristique de la variation du paramètre physiologique.

8. Système selon la revendication 7, **caractérisé en ce que** la présentation d'informations est interrompue lorsque la note est supérieure à un seuil prédéterminé.

9. Système selon la revendication 7, **caractérisé en ce qu'**il comprend une étape d'évaluation de la fiabilité de la recherche en comparant selon au moins un critère de ressemblance préétabli les informations ayant une note supérieure à un seuil prédéterminé.

10. Procédé de recherche d'informations dans une base de données informatique, **caractérisé en ce que** le procédé comprend les étapes de :
- présenter à un observateur au moins une des données et mesurer au moins un paramètre physiologique de l'observateur,
- en présence d'une variation du paramètre physiologique supérieur à un seuil prédéterminé, présenter une donnée ayant une relation de similarité avec la donnée précédemment présentée.

11. Procédé selon la revendication 10, **caractérisé en ce qu'**il comprend une étape préalable de calibration dans laquelle une mesure du paramètre physiologique est réalisée pour déterminer un niveau nominal de celui-ci.

12. Procédé selon la revendication 10, **caractérisé en ce que**, les données étant des images, le procédé comprend l'étape de détecter sur l'image au moins une partie d'intérêt pour l'observateur, la relation existant entre l'image précédemment présentée et l'image présentée après la détection de la variation du paramètre physiologique portant sur cette partie d'intérêt.

13. Procédé selon la revendication 12, **caractérisé en ce que** la partie d'intérêt est détectée en mesurant une durée d'examen de cette partie.

14. Procédé selon la revendication 12, **caractérisé en ce que** la partie d'intérêt est détectée en identifiant une corrélation entre une vision de cette partie et la variation du paramètre physiologique.

15. Procédé selon la revendication 12, **caractérisé en ce que** les images sont présentées par groupe et **en ce que** le procédé comprend l'étape de détecter une direction du regard de l'observateur lors de la présentation des images.

16. Procédé selon la revendication 10, **caractérisé en ce qu'**il comprend une étape de notation de l'information ayant déclenché la variation du paramètre physiologique au cours de laquelle est attribuée à l'information une note prenant en compte un degré d'attention de l'observateur pour le portrait et/ou une caractéristique de la variation du paramètre physiologique.

17. Procédé selon la revendication 16, **caractérisé en ce que** la présentation d'informations est interrompue lorsque la note est supérieure à un seuil prédéterminé.

18. Procédé selon la revendication 16, **caractérisé en ce qu'**il comprend une étape d'évaluation de la fiabilité de la recherche en comparant selon au moins un critère de ressemblance préétabli les informations ayant une note supérieure à un seuil prédéterminé.

## Claims

1. An information system comprising a server that includes calculation means, hosts a database, and is associated with data presentation means for presenting data from the database, and with means for measuring a physiological parameter of a user, the information system being arranged to implement a method of searching for information in a database, **characterized in that** the method being comprises the steps of:
• presenting at least one item of data to an observer and measuring at least one physiological parameter of the observer; and
• in the presence of a variation in the physiological parameter greater than a predetermined threshold, presenting an item of data having a relationship of similarity with the previously-presented data item that gave rise to the variation.

2. A system according to claim 1, **characterized in that** it includes a prior calibration step in which the physiological parameter is measured in order to determine a nominal level thereof.

3. A system according to claim 1, **characterized in that** for data items in the form of images, the method includes a step of detecting on an image at least one portion of interest to the observer, by means for detecting the viewing direction of the user, the relationship existing between the previously-presented image and the image presented after detecting a variation in the physiological parameter relating to said portion of interest.

4. A system according to claim 3, **characterized in that** the portion of interest is detected by measuring the length of time said portion is examined.

5. A system according to claim 3, **characterized in that** the portion of interest is detected by identifying a correlation between viewing said portion and the variation in the physiological parameter.

6. A system according to claim 3, **characterized in that** the images are presented in groups, and **in that** the method includes a step of detecting the observer's viewing direction while the images are being presented.

7. A system according to claim 1, **characterized in that** it includes a step of scoring the information that triggered the variation in the physiological parameter, during which the information is given a score that takes account of the degree of the observer's attention for the portrait and/or a characteristic of the variation in the physiological parameter.

8. A system according to claim 7, **characterized in that** the presentation of information is interrupted when the score is greater than a predetermined threshold.

9. A system according to claim 7, **characterized in that** it includes a step of evaluating the reliability of the search by using at least one pre-established resemblance criterion to compare the items of information that have a score greater than a predetermined threshold.

10. A method of searching for information in a computer database, the method being **characterized in that** it comprises the steps of:
• presenting at least one item of data to an observer and measuring at least one physiological parameter of the observer; and
• in the presence of a variation in the physiological parameter greater than a predetermined threshold, presenting an item of data having a relationship of similarity with the previously-presented data item.

11. A method according to claim 10, **characterized in that** it includes a prior calibration step in which the physiological parameter is measured in order to determine a nominal level thereof.

12. A method according to claim 10, **characterized in that** for data items in the form of images, the method includes a step of detecting on an image at least one portion of interest to the observer, the relationship existing between the previously-presented image and the image presented after detecting a variation in the physiological parameter relating to said portion of interest.

13. A method according to claim 12, **characterized in that** the portion of interest is detected by measuring the length of time said portion is examined.

14. A method according to claim 12, **characterized in that** the portion of interest is detected by identifying a correlation between viewing said portion and the variation in the physiological parameter.

15. A method according to claim 12, **characterized in that** the images are presented in groups, and **in that** the method includes a step of detecting the observer's viewing direction while the images are being presented.

16. A method according to claim 10, **characterized in that** it includes a step of scoring the information that triggered the variation in the physiological parameter, during which the information is given a score that takes account of the degree of the observer's attention for the portrait and/or a characteristic of the variation in the physiological parameter.

17. A method according to claim 16, **characterized in that** the presentation of information is interrupted when the score is greater than a predetermined threshold.

18. A method according to claim 16, **characterized in that** it includes a step of evaluating the reliability of the search by using at least one pre-established resemblance criterion to compare the items of information that have a score greater than a predetermined threshold.

## Patentansprüche

1. Datenverarbeitungssystem, umfassend einen Server, der Rechenmittel umfasst, eine Datenbank enthält und mit Mitteln zum Anzeigen von Daten aus der Datenbank und mit einem Mittel zum Messen eines physiologischen Parameters eines Benutzers verbunden ist, wobei das Datenverarbeitungssystem derart ausgebildet ist, dass es ein Verfahren zur Suche von Informationen in der Datenbank einsetzt, **dadurch gekennzeichnet, dass** das Verfahren die Schritte umfasst:
- einem Betrachter wird mindestens ein Datenelement der Daten angezeigt und mindestens ein physiologischer Parameters des Betrachters wird gemessen,
- bei Vorhandensein einer Variation des physiologischen Parameters über einen vorgegebenen Schwellenwert, wird ein Datenelement angezeigt, das eine Ähnlichkeitsrelation mit dem zuvor am Anfang der Variation angezeigten Datenelement hat.

2. System nach Anspruch 1, **dadurch gekennzeichnet, dass** es einen vorangehenden Kalibrierungsschritt umfasst, bei dem eine Messung des physiologischen Parameters erfolgt, um ein Nominalniveau desselben zu bestimmen.

3. System nach Anspruch 1, **dadurch gekennzeichnet, dass**, wenn die Daten Bilder sind, das Verfahren den Schritt umfasst, dass in dem Bild mindestens ein Areal von Interesse für den Betrachter durch eine Erfassungsvorrichtung zum Erfassen des Blickes des Betrachters erfasst wird, wobei die Relation zwischen dem zuvor angezeigten Bild und dem Bild besteht, das nach dem Erfassen der Variation des physiologischen Parameters angezeigt wird, der dieses Areal von Interesse betrifft.

4. System nach Anspruch 3, **dadurch gekennzeichnet, dass** das Areal von Interesse dadurch erfasst wird, dass eine Musterungsdauer dieses Areals gemessen wird.

5. System nach Anspruch 3, **dadurch gekennzeichnet, dass** das Areal von Interesse dadurch erfasst wird, dass eine Korrelation zwischen einem Sehen dieses Areals und der Variation des physiologischen Parameters identifiziert wird.

6. System nach Anspruch 3, **dadurch gekennzeichnet, dass** die Bilder gruppenweise angezeigt werden und dass das Verfahren den Schritt des Erfassens einer Richtung des Blickes des Betrachters während des Anzeigens der Bilder umfasst.

7. System nach Anspruch 1, **dadurch gekennzeichnet, dass** es den Schritt der Beurteilung der Information umfasst, die die Variation des physiologischen Parameters ausgelöst hat, während dem der Information eine Bewertung zugeordnet wird, die einen Aufmerksamkeitsgrad des Betrachters für das Portrait und/oder eine Charakteristik der Variation des physiologischen Parameters berücksichtigt.

8. System nach Anspruch 7, **dadurch gekennzeichnet, dass** die Informationsanzeige unterbrochen wird, wenn die Bewertung über einem vorgegebenen Schwellenwert liegt.

9. System nach Anspruch 7, **dadurch gekennzeichnet, dass** es einen Schritt der Evaluierung der Zuverlässigkeit der Suche umfasst, indem gemäß mindestens einem zuvor festgelegten Ähnlichkeitskriterium die Informationen verglichen werden, die eine Bewertung über einem vorgegebenen Schwellenwert haben.

10. Verfahren zur Suche von Informationen in einer Computerdatenbank, **dadurch gekennzeichnet, dass** das Verfahren die Schritte umfasst:
- mindestens ein Datenelement der Daten wird einem Betrachter angezeigt und mindestens ein physiologischer Parameter des Betrachters wird gemessen,
- bei Vorhandensein einer Variation des physiologischen Parameters über einen vorgegebenen Schwellenwert, wird ein Datenelement angezeigt, das eine Ähnlichkeitsrelation mit dem zuvor angezeigten Datenelement hat.

11. Verfahren nach Anspruch 10, **dadurch gekennzeichnet, dass** es einen vorangehenden Kalibrierungsschritt umfasst, bei dem eine Messung des physiologischen Parameters erfolgt, um ein Nominalniveau desselben zu bestimmen.

12. Verfahren nach Anspruch 10, **dadurch gekennzeichnet, dass**, wenn die Daten Bilder sind, das Verfahren den Schritt umfasst, dass in dem Bild mindestens ein Areal von Interesse für den Betrachter erfasst wird, wobei die Relation zwischen dem zuvor angezeigten Bild und dem Bild besteht, das nach dem Erfassen der Variation des physiologischen Parameters angezeigt wird, der dieses Areal von Interesse betrifft.

13. Verfahren nach Anspruch 12, **dadurch gekennzeichnet, dass** das Areal von Interesse dadurch erfasst wird, dass eine Musterungsdauer dieses Areals gemessen wird.

14. Verfahren nach Anspruch 12, **dadurch gekennzeichnet, dass** das Areal von Interesse dadurch erfasst wird, dass eine Korrelation zwischen einem Sehen dieses Areals und der Variation des physiologischen Parameters identifiziert wird.

15. Verfahren nach Anspruch 12, **dadurch gekennzeichnet, dass** die Bilder gruppenweise angezeigt werden und dass das Verfahren den Schritt des Erfassens einer Richtung des Blickes des Betrachters während des Anzeigens der Bilder umfasst.

16. Verfahren nach Anspruch 10, **dadurch gekennzeichnet, dass** es einen Schritt der Beurteilung der Information umfasst, die die Variation des physiologischen Parameters ausgelöst hat, während dem der Information eine Bewertung zugeordnet wird, die einen Aufmerksamkeitsgrad des Betrachters für das Portrait und/oder eine Charakteristik der Variation des physiologischen Parameters berücksichtigt.

17. Verfahren nach Anspruch 16, **dadurch gekennzeichnet, dass** die Informationsanzeige unterbrochen wird, wenn die Bewertung über einem vorgegebenen Schwellenwert liegt.

18. Verfahren nach Anspruch 16, **dadurch gekennzeichnet, dass** es einen Schritt der Evaluierung der Zuverlässigkeit der Suche umfasst, indem gemäß mindestens einem zuvor festgelegten Ähnlichkeitskriterium die Informationen verglichen werden, die eine Bewertung über einem vorgegebenen Schwellenwert haben.
